# EUROPÄISCHE PATENTSCHRIFT

(11) **EP 0 943 314 B1**
(45) Veröffentlichungstag und Bekanntmachung des Hinweises auf die Patenterteilung: **11.04.2001**
(21) Anmeldenummer: 99103603.9
(22) Anmeldetag: 24.02.1999
(51) Int. Cl.: A61K 7/06, A61K 7/13

(54) **Verwendung eines Mittels zur Haarbehandlung**
Use of a hair treatment composition
Utilisation d' une composition traitante pour la chevelure

(30) Priorität: 09.03.1998 DE 19810120
(43) Veröffentlichungstag der Anmeldung: 22.09.1999
(73) Patentinhaber: GOLDWELL GmbH, 64297 Darmstadt (DE)
(72) Erfinder: Fath, Bettina, 69469 Weinheim (DE)

(56) Entgegenhaltungen:
- EP-A- 0 750 900
- DE-A- 3 320 539
- DE-A- 4 432 549
- DE-C- 4 416 927
- DATABASE WPI Section Ch, Week 199339 Derwent Publications Ltd., London, GB; Class A96, AN 1993-309104 XP002126911 & JP 05 221838 A (ANPREIN KK; RACINE KAGAKU KK), 31. August 1993 (1993-08-31)
- DATABASE WPI Section Ch, Week 198548 Derwent Publications Ltd., London, GB; Class A96, AN 1985-298623 XP002126912 & JP 59 187097 A (KAO CORP), 24. Oktober 1984 (1984-10-24)

## Beschreibung

Die vorliegende Erfindung betrifft die Verwendung eines Mittels, insbesondere eines Haarkonditioniermittels, das dem menschlichen Haar verbesserte Naß- und Trockenkämmbarkeit, Fülle und Halt sowie Glanz verleiht.

Mittel zum Konditionieren von menschlichen Haaren sind seit langem bekannt.
Sie enthalten in der Regel quaternäre Ammoniumverbindungen, die mindestens eine langkettige Alkyl- oder Alkenylgruppe aufweisen, und gegebenenfalls auch Polymere.

Solche Mittel werden üblicherweise als wäßrige Dispersionen bzw. Emulsionen, Mikroemulsionen, Gele oder auch in Aerosolform konfektioniert und als Haarspülungen, Kuren, etc. eingesetzt.

Eine Übersicht über die bekannten Haarnachbehandlungsmittel und ihre Zusammensetzung, die nach der Anwendung als "Rinse-off" Produkte aus dem Haar ausgespült werden oder aber als "Leave-on" Produkte im Haar verbleiben, findet sich in der Monographie von K. Schrader, Grundlagen und Rezepturen der Kosmetika, 2. Aufl. (1989), S. 722 bis 781, insbesondere S. 728 bis 737.

Trotz dieser bekannten Zusammensetzungen besteht immer noch ein Bedürfnis nach Haarbehandlungsmitteln mit verbesserten haarpflegenden Eigenschaften.

Es wurde nunmehr gefunden, daß ein Haarbehandlungsmittel, das bei topischer Anwendung die Eigenschaften des Haares verbessert, dann erhalten wird, wenn man einem solchen Mittel auf wäßriger Basis eine Mischung aus mindestens einem Mono- und/oder Oligosaccharid und Grünen Tee- Extrakt zusetzt.

Als Mono- und/oder Oligosaccharide in den erfindungsgemäß verwendeten Haarbehandlungsmitteln werden vorzugsweise Saccharose, Glucose, Fructose, Lactose, Maltose, Galactose, Cellobiose, Fucose, Mannose, Erythrose, Rhamnose, etc. eingesetzt.
Es ist zweckmäßig, diese Zucker als Gemische, beispielsweise als Bestandteile von Naturstoffen, zu verwenden.
Ein bevorzugter, Mono- und Disaccharide enthaltender Naturstoff ist beispielsweise Honig.
Auch Stärkehydrolysate sind geeignet.
Der Anteil in der Gesamtzusammensetzung bezieht sich dabei jeweils auf den Mono- bzw. Oligosaccharidanteil.
Geeignete Mengen sind etwa 0,1 bis 20 Gew.-%, insbesondere 0,25 bis 10 Gew.-%.
Besonders bevorzugt sind dabei 0,5 bis 5 Gew.-% der Gesamtzusammensetzung.

Der eingesetzte pulvrige Tee-Extrakt wird aus Blättern, Blattknospen und zarten Stielen des Teestrauchs, Camellia sinensis bzw. Camellia oleifera, durch wäßrige bzw. wäßrigalkoholische Extraktion und anschließende Sprühtrocknung erhalten.

Bei grünem Tee handelt es sich um die aus den Arten Thea sinensis bzw. Thea assamica gewonnenen, im Gegensatz zum schwarzen Tee nicht fermentierten Produkte.

Eine Übersicht über die biologische und pharmakologische Wirkung grünen Tees sowie seine Inhaltsstoffe findet sich z.B. in einem Artikel von A. Pistorius, SÖFW-Journal, 122. Jahrgang, No. 7/1996, S. 468-471, auf den Bezug genommen wird.

Der Gehalt an Extrakt aus grünem Tee in den erfindungsgemäß verwendeten Zusammensetzungen ist variabel. Er liegt bei mindestens 0,1, vorzugsweise bei 0,1 bis 10, vorzugsweise 0,25 bis 5, insbesondere 0,5 bis 2,5 Gew.-%, berechnet als Trockenmasse auf die Gesamtzusammensetzung des Mittels.
Die Verwendung von Flavanol-Derivaten, die u.a. aus grünem Tee gewonnen werden können, in Haarpflegemitteln ist bereits aus der DE 33 20 539 A1 bekannt.
Die erfindungsgemäß verwendete Kombination ist dieser Druckschrift nicht zu entnehmen.

Die erfindungsgemäß verwendeten Haarbehandlungsmittel können natürlich zusätzlich die in solchen Mitteln üblichen Bestandteile enthalten; es wird, zur Vermeidung von Wiederholungen, wiederum auf K. Schrader, "Grundlagen und Rezepturen der Kosmetika", 2. Aufl. (1989), S. 722-771, verwiesen.

Geeignete Zusatzstoffe sind die in solchen Zusammensetzungen an sich bekannten quaternären Ammoniumverbindungen mit einer oder zwei Alkyl- bzw. Alkenylgruppen mit 10 bis 22 Kohlenstoffatomen im Molekül, insbesondere in einer Menge von 0,1 bis 10, vorzugsweise 0,25 bis 7,5, besonders bevorzugt 0,5 bis 5 Gew.-%, der Gesamtzusammensetzung.

Geeignete langkettige quaternäre Ammoniumverbindungen, die allein oder im Gemisch miteinander eingesetzt werden können, sind insbesondere Cetyltrimethylammoniumchlorid, Dimethyldicetylammoniumchlorid, Trimethylcetylammoniumbromid, Behenyltrimoniumchlorid, Stearyltrimethylammoniumchlorid, Dimethylstearylbenzylammoniumchlorid, Benzyltetradecyldimethylammoniumchlorid, Dimethyl- dihydriertes-Talgammoniumchlorid, Laurylpyridiniumchlorid, Lauryldimethylbenzylammoniumchlorid, Lauryltrimethylammoniumchlorid, Tris(oligooxyethyl)alkylammoniumphosphat, Cetylpyridiniumchlorid, etc. Grundsätzlich sind alle quatemären Ammoniumverbindungen geeignet, die im CTFA International Cosmetic Ingredient Dictionary unter der Bezeichnung "Quaternium" aufgeführt sind.

Vorzüglich geeignet sind auch langkettige Ammoniumverbindungen mit einer oder zwei Estergruppen im Molekül, die sogenannten Esterquats.

Diese entsprechen vorzugsweise der allgemeinen Formel 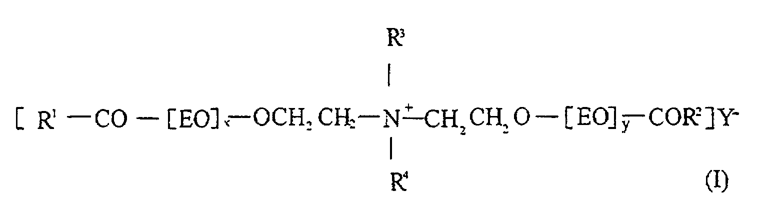 in der R¹ und R² jeweils für eine gegebenenfalls hydroxysubstituierte C₈-C₂₂- Alkyl- oder Alkenylgruppe, R³ und R⁴ für eine C₁-C₃-Alkylgruppe oder eine Gruppe -CH₂-CH₂-O-[EO]_{z}-H, sowie x,y und z für 0 bis 5, und Y⁻ für ein Anion stehen.

Bevorzugte Reste R¹ und R² sind C₁₂-C₁₈-Alkyl- und Oleylreste, der Rest R³ ist eine Methylgruppe, und der Rest R⁴ eine Hydroxyethylgruppe, x,y und z sind vorzugsweise 0 oder 1; Y⁻ ist vorzugsweise ein Methosulfat-, Chlorid- oder Phosphatanion.

Die bevorzugte Menge an dieser Verbindung der Formel I liegt bei etwa 0,1 bis etwa 20, insbesondere etwa 0,5 bis 15, vor allem etwa 1 bis etwa 10 Gew.-%, berechnet auf das Haarbehandlungsmittel.

Diese Verbindungen sind an sich bekant und beispielsweise unter den Markenbezeichnungen "Schercoquat^{R}", "Dehquart^{R}F30" und "Tetranyl^{R}" im Handel.

Ihre Verwendung in Haarpflegemitteln ist insbesondere in der WO 94/06899 A, der EP 0 614 349 B1 und der 0 680 314 B1 beschrieben.

Weitere Zusatzstoffe sind beispielsweise synthetische oder natürliche haarkonditionierende Polymere, vorzugsweise in einer Menge von 0,1 bis 2,5, insbesondere 0,25 bis 1,5 Gew.-% der Gesamtzusammensetzung.

Besonders bevorzugt sind hierbei die unter der CTFA-Bezeichnung "Polyquaternium" bekannten kationischen (Co-)Polymeren, alleine oder auch im Gemisch mit nichtionischen, anionischen und/oder amphoteren Polymeren, beispielsweise solchen vom Typ "Amphomer^{R}".

Geeignete kationische Polymere sind neben den altbekannten quaternären Cellulosederivaten des Typs "Polymer^{®} JR" insbesondere quaternisierte Homo- und Copolymere des Dimethyldiallylammoniumchlorids, wie sie unter dem Handelsnamen "Merquat^{®}" im Handel sind, quaternäre Vinylpyrrolidon-Copolymere, insbesondere mit Dialkylaminoalkyl(meth)acrylaten, wie sie unter dem Namen "Gafquat^{®}" bekannt sind, Copolymerisate aus Vinylpyrrolidon und Vinylimidazoliniummethochlorid, die unter dem Handelsnamen "Luviquat^{®}" angeboten werden, Polyamino-Polyamid-Derivate, beispielsweise Copolymere von Adipinsäure-Dimethylaminohydroxypropyldiethylentriamin, wie sie unter dem Namen "Cartaretine^{®} F" vertrieben werden, sowie auch bisquaternäre langkettige Ammoniumverbindungen der in der US-A 4 157 388 beschriebenen Harnstoff-Strutur, die unter dem Handelsnamen "Mirapol^{®} A 15" im Handel sind.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Gut geeignete kationische Polymere sind auch die in der EP-A 640 643 geoffenbarten Pfropfcopolymerisate aus Organopolysiloxanen und Polyethyloxazolin.
Ein besonders bevorzugtes Pfropfcopolymerisat der dargestellten Art weist ein Gesamtmolekulargewicht von etwa 50 000 bis etwa 500 000, vorzugsweise etwa 80 000 bis etwa 300 000, insbesondere etwa 100 000 Dalton auf, wobei das Molgewicht des Oxazolin-Segments etwa 2 500 bis etwa 7 500, vorzugsweise etwa 4 000 bis etwa 6 000, insbesondere etwa 5 000 Dalton-Segment beträgt, d.h. dessen Molanteil bei 20 Einheiten/Molekül liegt. Der bevorzugte Si-Gehalt beträgt, entsprechend der Elementaranalyse, etwa 50%.
Besonders geeignet sind die unter den Bezeichnungen A-1, A-2 und A3 auf den Seiten 12 bis 13 der EP-A 640 643 beschriebenen Organopolysiloxane.

Gemäß einer weiteren Ausführungsform der Erfindung kannn das Haarbehandlungsmittel noch mindestens eine Verbindung, ausgewählt aus der Gruppe 1-Methoxypropanol(-2), 1-Ethoxypropanol(-2), Diethylenglykolmonomethyl- oder -ethylether, Dipropylenglykolmonomethyl- oder -ethylether, Benzylalkohol, Benzyloxyethanol, Phenylethylalkohol, Phenoxyethanol und/oder Zimtalkohol, vorzugsweise in einer Menge von 0,1 bis 20, insbesondere 0,5 bis 15, vor allem 1 bis 12,5 und 2,5 bis 10 Gew.-%, berechnet auf die Geamtzusammensetzung des Mittels enthalten. Bevorzugte Verbindungen aus dieser Gruppe sind Ethoxydiglykol und Benzyloxyethanol.

Weitere Zusatzstoffe, deren Art und Menge natürlich von der Applikationsform des Mittels abhängig ist, sind Fette, Fettalkohole, Emulgatoren, pH-Regulatoren, Lösungs- und Verdünnungsmittel, Lösungsvermittler, Konservierungsmittel, Farbstoffe, Parfums, etc.

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven-bzw. Sojaöl, Lanolin und dessen Derivate, ebenso Mineralöle wie Paraffinöl und Vaseline.

Synthetische Öle und Wachse sind beispielsweise Silikonöle, Polyethylengykole, etc. Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethlenglykol- und Polyglycerylfettsäureester wie PEG-7-glycerylcocoat, Cetylpalmitat, etc.

Diese hydrophoben Komponenten sind in der erfindungsgemäßen Zusammensetzungen vorzugsweise in einer Gesamtmenge von etwa 0,5 bis etwa 10, insbesondere etwa 1 bis 7,5, vorallem etwa 1,5 bis 5 Gew.-%, berechnet auf die Gesamtzusammensetzung, enthalten.

Ebenso können neben den oben erwähnten quaternären langkettigen Ammoniumverbindungen auch andere oberflächenaktive Stoffe, insbesondere amphotere (zwitterionische) und/oder nichtionische Tenside, deren einschlägige Verwendung natürlich an sich bekannt ist, eingesetzt werden.

Eine beispielhafte Zusammenfassung der Herstellung solcher Mittel findet sich ebenfalls in der bereits erwähnten Monographie von K. Schrader, S. 798 bis 818, insbesondere S. 804 ff.

Die erfindungsgemäß verwendeten haarkonditionierenden Mittel liegen vorzugsweise als wäßrige Emulsion, Mikroemulsion, Dispersion oder opakes oder transparentes Gel vor, und können auch als Aerosole konfektioniert werden. Solche Zusammensetzungen und ihre Herstellung sind dem Fachmann grundsätzlich bekannt und bedürfen daher keiner näheren Erläuterung.

Eine bevorzugte Anwendungsform sind Mittel, enthaltend anionische oder kationische Farbstoffe; es hat sich gezeigt, daß die erfindungsgemäße Kombination auch eine farbstabilisierende Wirkung auf gefärbte Produkte ausübt.

Der pH-Wert der erfmdungsgemäß verwendeten Haarbehandlungsmittel ist nicht kritisch; er kann vorzugsweise bei 3 bis etwa 8, insbesondere zwischen 4 und 6,5, liegen.

Die folgenden Beispiele dienen der Illustration der Erfindung.

### Beispiel 1

In eine Haar-Aufbaukur der Zusammensetzung I

| | |
|---|---|
| 2,5 (Gew.-%) | Cetrimoniumchlorid |
| 0,6 | Di-C₁₂-C₁₅-alkyldimoniumchlorid |
| 6,0 | Behensäure |
| 3,5 | Benzyloxyethanol |
| 5,0 | Ethoxydiglykol |
| 0,5 | Hydroxyethylcellulose |
| 0,2 | Polyquaternium-4 |
| 35,0 | Glycerin |
| 0,5 | Saccharose |
| 0,5 | Isopropylpalmitat |
| 1,0 | PEG-30 Steareth-4 |
| 2,0 | Stearinsäure |
| 5,0 | Dimethicone |
| 1,0 | Dimethicone Methylether |
| 0,3 | Parfum |
| 0,5 | Natriumhydroxid |
| ad 100,00 | Wasser |

wurden, unter entsprechender Verringerung des Wassergehalts, 0,5 Gew.-% Grüner Tee-Extrakt eingearbeitet (Zusammensetzung I A).

Beide Zusammensetzungen wurden im Halbseitenversuch auf je eine Hälfte dauergewellten Haares aufgebracht und nach dem Einarbeiten und Trocknen die Eigenschaften beider Haarhälften verglichen.

Die mit der erfindungsgemäßen Zusammensetzung I A behandelte Haarhälfte wies gegenüber der mit der Haar-Aufbaukur I behandelten Haarhälfte eine deutlich verbesserte Naß- und Trockenkämmbarkeit, höheren Glanz, besseren Halt und Körper auf.

### Beispiel 2

In eine Emulsion der Zusammensetzung II

| | |
|---|---|
| 3,0 (Gew.-%) | Cetrimoniumchlorid |
| 0,3 | Talgtrimoniumchlorid |
| 2,0 | Benzyloxyethanol |
| 10,0 | Ethoxydiglykol |
| 6,0 | Behensäure |
| 0,7 | Glykolsäure |
| 0,4 | Hydroxyethylcellulose |
| 0,3 | Isostearylpentaerythrylglycerylether |
| 30,0 | Glycerin |
| 0,5 | Grüner Tee-Extrakt |
| 0,3 | Polyquaternium-7 |
| 1,5 | PEG-20 Steareth-4 |
| 0,4 | Trideceth-12 |
| 2,0 | Stearinsäure |
| 0,5 | Parfum |
| 0,4 | Dimethiconol |
| 0,3 | Amodimethicone |
| 1,2 | Natriumhydroxid |
| 0,3 | Weizenkeimöl |
| ad 100,0 | Wasser |

wurden 0,8 Gew.-% Saccharose unter entsprechender Verringerung des Wassergehalts eingearbeitet (Zusammensetzung II A).
Es wurde im Halbseitenversuch mit der Zusammensetzung II A eine gegenüber der Zusammensetzung II deutlich verbesserte Konditionierwirkung erzielt.

### Beispiel 3

| **Leave-on Lotion** | |
|---|---|
| Distearyldimethylammoniumchlorid | 0,6 (Gew.-%) |
| Amodimethicone | 2,0 |
| Aminosäuren (L-Arginin+D-Glucosamin.HCl) | 0,5 |
| Polyquaternium-11 | 0,3 |
| Pyrrolidon-2-carbonsäure, Natriumsalz | 0,2 |
| Citronensäure | 0,2 |
| Milchsäure | 0,1 |
| Glyoxylsäure | 0,5 |
| Saccharose | 0,3 |
| Grüner Tee-Extrakt | 0,5 |
| Fructose | 0,3 |
| PEG-40 Hydriertes Ricinusöl | 0,3 |
| Panthenol | 1,0 |
| Tocopherolacetat | 0,1 |
| Parfum | 0,3 |
| Konservierungsmittel | 0,3 |
| Wasser | ad 100,00 |

### Beispiel 4

| **Haarspülung** | |
|---|---|
| Cetylstearylalkohol | 1,00 |
| Mandelöl | 0,50 |
| PEG-7 Glycerylcocoat | 0,50 |
| Hydroxyethylcellulose | 1,00 |
| Saccharose | 0,50 |
| Benzophenone-4 | 0,30 |
| Grüner Tee-Extrakt | 0,50 |
| Dimethicone Copolyol Beeswax | 0,80 |
| Decylglucosid | 0,50 |
| 1,2-Propylenglykol | 1,00 |
| Dimethicone | 0,20 |
| Behentrimoniumchlorid | 0,40 |
| Parfum | 0,30 |
| Konservierungsmittel (Parabene) | 0,20 |
| Gelber Farbstoff C.O.-No. 47,005 | 0,0003 |
| Roter Farbstoff C.O.-No. 14,700 | 0,0009 |
| Wasser | ad 100,00 |

### Vergleichstest

Eine Zusammensetzung IV nach Beispiel 4 wurde an 10 Probanden im Halbseitentest auf eine Hälfte des Haares aufgebracht und im Vergleich zur anderen Haarhälfte, die mit einer ansonsten identischen Zusammensetzung IVA, die anstelle der Saccharose den gleichen Anteil Behentrimethylammoniumchlorid enthielt, behandelt worden war, die folgenden Parameter nach einer einfachen Gut-gleich-schlechter Bewertung von 2 Friseuren manuell und visuell bestimmt.

| **Ergebnis** | | | |
|---|---|---|---|
| **Präferenz** | **Zusammensetzung IV** | **Gleich** | **Zusammensetzung IV A** |
| Naßkämmbarkeit | 14 | 4 | 2 |
| Naßgriff | 11 | 8 | 1 |
| Trockenkämmbarkeit | 15 | 3 | 2 |
| Trockengriff | 16 | 4 | 0 |
| Fülle (Volumen, Body) | 13 | 3 | 3 |
| Glanz | 18 | 1 | 1 |

Daraus ergibt sich eine eindeutige Präferenz für die erfindungsgemäße Zusammensetzung.

Gleichzeitig wurde ein Farbstabilitätstest bei Raumtemperatur durchgeführt, wobei die Original-Zusammensetzung IV nach Beispiel 4 mit einer ansonsten identischen Zusammensetzung IV A, die jedoch keine Saccharose und keinen Grünen Tee-Extrakt enthielt, nach ein-, zwei- und dreimonatiger Lagerung verglichen wurde.

Dabei zeigte sich, daß bereits nach einmonatiger Lagerung ein sichtbarer Unterschied feststellbar war, nach 3 Monaten war die Farbe der Zusammensetzung IV A deutlich ausgeblaßt gegenüber derjenigen der Zusammensetzung IV, die sich damit als deutlich farbstabiler erwies.

## Patentansprüche

1. Verwendung eines Mittels auf wäßriger Basis, enthaltend
a) mindestens etwa 0,1 Gew.-% mindestens eines Mono- und/oder Oligosaccharids, und
b) mindestens 0,1 Gew.-%, berechnet als Trockenmasse auf die Gesamtzusammensetzung des Mittels, Grünen Tee-Extrakt,
zur Haarbehandlung.

2. Verwendung eines Mittels nach Anspruch 1, enthaltend 0,1 bis 10 Gew.-% Grünen Tee-Extrakt, berechnet als Trockenmasse auf die Gesamtzusammensetzung des Mittels.

3. Verwendung eines Mittels nach Anspruch 2, enthaltend 0,25 bis 5,0 Gew.-% Grünen Tee-Extrakt, berechnet als Trockenmasse auf die Gesamtzusammensetzung des Mittels.

4. Verwendung eines Mittels nach einem oder mehreren der Ansprüche 1 bis 3, enthaltend 0,1 bis 10 Gew.-% mindestens eines Mono- und/oder Oligosaccharids.

5. Verwendung eines Mittels nach einem oder mehreren der Ansprüche 1 bis 4, enthaltend ein kationisches Polymer.

6. Verwendung eines Mittels nach einem oder mehreren der Ansprüche 1 bis 5, enthaltend mindestens eine quaternäre Ammoniumverbindung mit mindestens einer C₁₀-C₂₂-Alkyl- oder Alkenylgruppe.

7. Verwendung eines Mittels nach einem oder mehreren der Ansprüche 1 bis 6, enthaltend mindestens einen anionischen oder kationischen Farbstoff.

## Claims

1. Use of a Composition on aqueous basis, comprising
a) at least about 0.1% by weight of a mono- and/or oligosaccharide, and
b) at least 0.1% by weight green tea extract, calculated as dry content to the total composition,
for the treatment of human hair.

2. Use of a Composition according to claim 1, comprising 0.1% to 10 % by weight green tea extract, calculated as dry content to the total composition.

3. Use of a Composition according to claim 2, comprising 0.25% to 5.0 % by weight green tea extract, calculated as dry content to the total composition.

4. Use of a Composition according to one or more of claims 1 to 3, comprising 0.1% to 10% by weight of at least one mono- and/or oligosaccharide.

5. Use of a Composition according to one or more of claims 1 to 4, comprising a cationic polymer.

6. Use of a Composition according to one or more of claims 1 to 5, comprising at least one quaternary ammonium compound with at least one C₁₀-C₂₂-alkyl or alkenyl group.

7. Use of a Composition according to one or more of claims 1 to 6, comprising at least one anionic or cationic dyestuff.

## Revendications

1. Utilisation d'une composition à base aqueuse, contenant
a) au moins environ 0,1% en poids d'au moins un monosaccharide et/ou oligosaccharide, et
b) au moins 0,1% en poids, en matière sèche, rapporté à la composition totale de la composition, d'extrait de thé vert,
pour le traitement des cheveux.

2. Utilisation d'une composition selon la revendication 1, contenant 0,1 à 10% en poids d'extrait de thé vert, rapporté, en matière sèche, à la composition totale de la composition.

3. Utilisation d'une composition selon la revendication 2, contenant 0,25 à 5,0% en poids d'extrait de thé vert, rapporté, en matière sèche, à la composition totale de la composition.

4. Utilisation d'une composition selon l'une ou plusieurs des revendications 1 à 3, contenant 0,1 à 10% en poids d'au moins un monosaccharide et/ou oligosaccharide.

5. Utilisation d'une composition selon l'une ou plusieurs des revendications 1 à 4, contenant un polymère cationique.

6. Utilisation d'une composition selon l'une ou plusieurs des revendications 1 à 5, contenant au moins un composé ammonium quaternaire ayant au moins un groupe alkyle ou alcényle en C₁₀-C₂₂.

7. Utilisation d'une composition selon l'une ou plusieurs des revendications 1 à 6, contenant au moins un colorant anionique ou cationique.
